Europäisches Patentamt

European Patent Office

Office européen des brevets

(11) Publication number: **0 058 857**
**B1**

(12) **EUROPEAN PATENT SPECIFICATION**

(45) Date of publication of patent specification: **04.06.86**

(51) Int. Cl.⁴: **A 61 K 37/02, C 12 P 21/00**

(21) Application number: **82100868.7**

(22) Date of filing: **06.02.82**

(54) Hepatosin, process for preparing it and therapeutical composition containing hepatosin.

(30) Priority: **24.02.81 US 237729**

(43) Date of publication of application:
**01.09.82 Bulletin 82/35**

(45) Publication of the grant of the patent:
**04.06.86 Bulletin 86/23**

(84) Designated Contracting States:
**AT BE CH DE FR GB IT LI LU NL SE**

(56) References cited:
US-A-3 876 774
US-A-4 209 587

CHEMICAL ABSTRACTS, vol. 74, 1971, page
306, no. 138406w, Columbus, Ohio, USA, V.N.
FEDORICH et al.: "Modeling of autoallergic liver
disease"

(73) Proprietor: **Renoux, Gérard E.**
**1 bis, rue des Ursulines**
**F-37000 Tours (FR)**
(73) Proprietor: **Renoux, Micheline J.**
**1 bis, rue des Ursulines**
**F-37000 Tours (FR)**

(72) Inventor: **Renoux, Gérard E.**
**1 bis, rue des Ursulines**
**F-37000 Tours (FR)**
Inventor: **Renoux, Micheline J.**
**1 bis, rue des Ursulines**
**F-37000 Tours (FR)**

(74) Representative: **Fiedler, Otto Karl, Dipl.-Ing.**
**Hug Interlizenz AG Alte Zürcherstrasse 49**
**CH-8903 Birmensdorf (CH)**

Courier Press, Leamington Spa, England.

# 0 058 857

**Description**

Background of the invention

Three general approaches have been used in recent times for drug development (1) screening large numbers of synthetic test compounds of diverse chemical structures (2) mimicking nature by designing compounds that exert an effect similar to a naturally occurring substance and (3) modifying the structure of an already known drug to maximize or improve one property or minimize an undesirable property. Classically, however, isolation of active principles from biological materials has been quite successful in producing therapeutically useful substances. The isolation of insulin from pancreas glands, guinine from Cinchona bark, and within the field of immunology, the isolation of polypeptide-like materials (Thymosin V, Thymopoietin) derived from the thymus gland which are able to restore or augment depressed immunity are a few examples. A method for restoration of depressed immunity can be medically useful in any disease in which a primary immunodeficiency exists or a secondary immunodeficiency is produced.

The discovery of hormones derived from the thymus and their use in medicine has recently become important and there is considerable literature thereon, for example:

Goldstein U.S. patent 4,002,740
Goldstein U.S. patent 4,079,127
DeSomer U.S. patent 3,438,859
Goldstein U.S. patent 4,082,737
Yeshiva British patent 1,195,980
Brunetti U.S. patent 3,657,417
Goldstein U.S. patent 4,002,602
Goldstein U.S. patent 4,077,949
Renoux, The Journal of Experimental Medicine, Vol. 145 (1977) pages 466—471

Summary of the invention

The object of the present invention is to propose a novel polypeptide-like substance called hepatosin, a method for preparing said hepatosin by isolation of the novel polypeptide-like substance which is not derived from the thymus and that can be produced by liver cells in culture and can be found in the serum of all species that can be used to enhance immunity in animals. Further an object of the invention is to provide a new theraupeutical composition for imparting immunomodulating activity, antiviral activity or anti-leukemic activity.

These objects are accomplished by providing hepatosin, being a polypeptide-like substance with a molecular weight between 500 and 5000 and having an amino acid composition of aspartic acid, threonine, glutamic acid, glycine, cystine, tyrosine, phenylalanine and lysine and being obtainable by culturing liver cells on the medium RPMI-1640 supplemented with L-glutamine and recovering the hepatosin thus prepared.

Further, the invention refers to a method of preparing said hepatosine comprising culturing liver cells on the medium RPMI-1640 supplemented with L-glutamine and recovering the hepatosin thus prepared. In an improved method the culture medium includes an immunopotentiator in an amount of $10^{-3}$ to $10^{-12}$ mM/ml whereby the sodium salt of diethyldithiocarbamic acid, levamisole or inosiplex are preferred.

Finally, the invention proposes a therapeutical composition containing hepatosin and especially in a concentration of at least about 100 times as great as it is obtained by culturing $C_3H$ mouse liver cells by suspending 50 million liver cells in 50 ml of RPMI-1640 supplemented with 170 L-glutamine at 37°C for 24 hours in an atmosphere of 95% air, 5% $CO_2$, followed by harvesting by centrifugation. Preferably the liquid composition contains the hepatosin in an amount of at least 40 mg/ml and is most preferably aqueous liquid composition.

The results presented herein demonstrate that (1) the novel biologically derived substance can be used to enhance cellular immunity (2) the origin of this substance is extra thymic (liver) and (3) assay of the material in serum can be used as a measure of immune competence. The new material is called hepatosin.

Brief description of the drawings

Figure 1 is a graph of the induction of T-cell Markers by hepatosin (identified as DMH) prepared in the presence of sodium diethyldithiocarbamate (DTC),

Figure 2 is a similar graph for the mean values for four batches of hepatosin (DMH); and

Figure 3 is a graph of the induction of T-cell Markers similar to Figure 1 but with hepatosin prepared in the absence of DTC.

Detailed description

Example 1

Preliminary assays revealed that when $C_3H$ mouse liver cells were cultured for 24 hours in the presence of the sodium diethyldithiocarbamate (DTC), a soluble factor (hormone) was produced which was able to increase the immune responsiveness of $C_3H/He$ female mice to immunization with sheep red cells (SRC). The preparation of this "differentiating and maturing hormone" (DMH) which is given the name hepatosin was carried out by a technique summarized as follows: Livers were aseptically removed by passage

2

through a sterile steel mesh. Fifty million liver cells were suspended in 50 ml of RPMI-1640 supplemented with 1% L-glutamine (But without fetal calf serum, antibiotics or phenol red) and $10^{-5}$ mM/ml (millimoles per ml) of DTC, and cultured in tissue culture flasks (#3075 Costar, Cambridge, Massachusetts). After 24 hours of incubation at 37°C in an atmosphere of 95% air, 5% $CO_2$ and saturated vapor, supernatants were harvested by centrifugation at 4°C. Aliquots were tested for trypan blue tests (more than 95% viable liver cells at the time of harvest), cultured on trypticase-soy agar and tested for *Limulus* assays to discard any butch evidencing bacterial contamination or measurable endotoxin-like activity. Supernatants were heated at 100°C for 1 hour, kept overnight at 4°C and spun in the cold in a refrigerated PR-J International Centrifuge. The clear supernatant was purified by concentration on an UM 05 membrane to discard salts, free amino acids and residual DTC. The active fraction, DMH, passed through a UM 5 membrane, thus indicating a molecular weight less than 5000. The concentrate contained 40 to 45 mg (dry weight) per ml and was filtered through a 0.43 µ membrane before use. The biological activity of this preparation of DMH is described below under "Biological Activity". Unless otherwise indicated the biological data was obtained on the product obtained using the $C_3H$ mouse liver cells.

Example 2
Production of DMH by liver cells of *nu/nu* mice (athymic)
Liver cells ($10^6$ cells) were cultured in media as in Example 1 in the presence of $10^{-5}$ mM of DTC per ml. The final product was obtained after centrifugation and filtration through UM5 membranes to discard salts, free amino acids, etc... and through a UM10 membrane (MW higher than 500, less than 10,000).

Example 3
The incubation was carried out in the absence of DTC. The procedure otherwise was as in Example 1. There was obtained a product having similar properties to DMH but in an amount equivalent to 1% of the level obtained using DTC.
Hepatosin has a molecular weight between 500 and 5000 as shown by ultrafiltration studies, is resistant to heating at 100°C and has an amino acid composition of aspartic acid, threonine, glutamic acid, glycine, cystine, tyrosine, phenylalanine and lysine.
In place of DTC to enhance the amount of hepatosin formed there can be used other immunopotentiators agents such as levamisole (The Merck Index 9th edition compound No. 8949) or Inosiplex (The Merck Index 9th edition compound No. 4853, available under the trademark Isoprinosine and described in Gordon U.S. patent 3,646,007.
The range in amount of DTC (or other immunopotentiator) employed with the medium for example can be from $10^{-3}$ to $10^{-12}$ mM/ml.
The incubation time is preferably 24 hours but can be varied, e.g. from 18 to 72 hours.
While incubation is preferably carried out at about 37°C it can be carried out at other temperatures, e.g., 20°C.
In Example 1 the aliquots were tested simply to make sure the cells were alive. The culturing was to make sure there was no bacterial contamination. The heating to 100°C was to eliminate chemical contaminants. Thus heating precipitates and denatures the contaminants.

Biological properties
Hepatosin (DMH) is a polypeptide material which is able to cause precursor cells destined to become T-cells to differentiate the T-cells and to mature to the extent of being able to possess the functional properties of a T-cell.

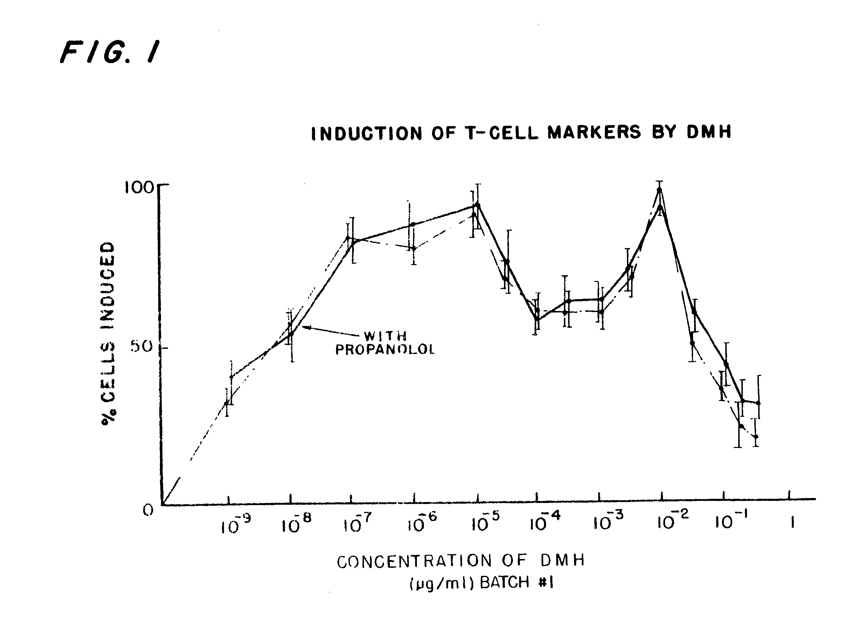

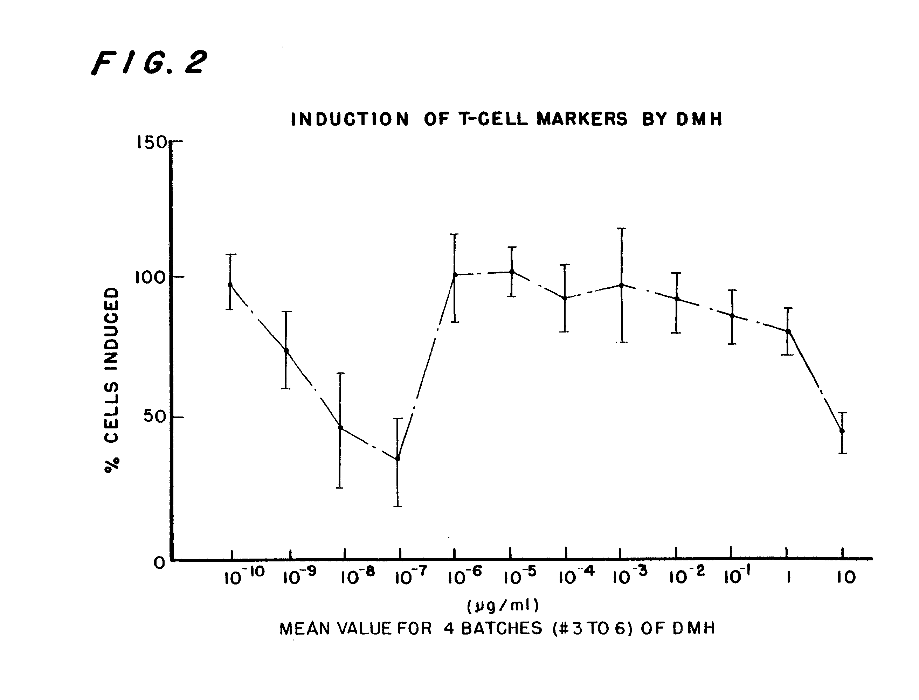

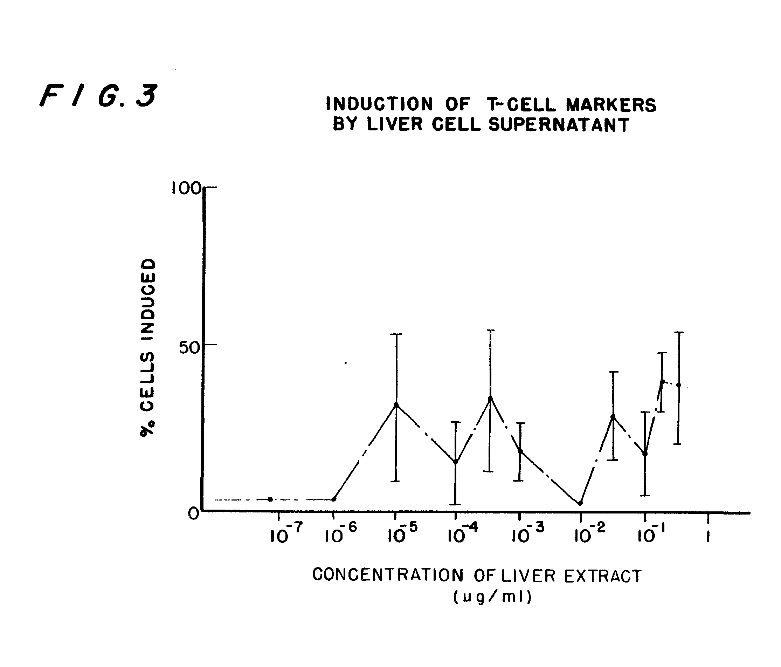

(A) Differentiation
1) In vitro
(a) Induction of Thy-1$^+$ marker on precursor spleen cells from nu/nu mice
Using the assay of Komuro-Boyse, $5 \times 10^6$ spleen cells isolated from *nu/nu* mice were incubated at 37°C in a 5% $CO_2$ atmosphere for 3.5 hours. The appearance of Thy-1$^+$ (T-cell marker) was measured at varying concentrations of DMH and was expressed as % of maximum induction varied between 20—26%. Induction of CR$^+$ (B-cell marker) was not seen upon incubation of *nu/nu* cells in DMH, and even after a 18 hr. incubation.
The data presented in Figure 1 demonstrated that maximal induction occurred at $10^{-7}$ µg/ml with significant induction occurring at $10^{-9}$ µg/ml. This activity was greater than that observed for the thymic hormones on a µg/ml basis. Propranolol (the Merck Index, 9th edition compound No. 7628) a B-adrenergic blocking agent did not prevent induction. The propranolol was used in an amount of $10^{-5}$ mM/ml. The continuous line curve was the one with propranolol.
The data shown in Figure 2 gave a somewhat different dose response curve and shows the mean values obtained for four batches of DMH. Activity was still seen at $10^{-9}$ µg/ml and maximal effects were noted at $10^{-6}$ µg/ml. The double peak dose response curve might signify that the DMH preparation was impure or could be evidence of a dual receptor system on the cell surface.
The data presented in Figure 3 was obtained from a liver culture supernatant (LCS) prepared in the same manner as DMH, but produced in the absence of DTC in the culture media. It is obvious that some

3

**0 058 857**

induction capacity existed in these cell supernatants, but the capacity was much less than when DTC was added.

(b) Induction of T-cell from precursor human peripheral blood null cells (acquisition of HTLA antigen)

To further test the hormone-like response of DMH, there was studied its effectiveness in recruiting T-cells from Null cells of human peripheral blood.

Human peripheral blood cells were purified on Ficoll-Hypaque density gradient and deprived of T-cells by two subsequent E rosetting -and Ficol-Hypaque gradients. After two washes with RPMI medium, the sedimented pellet was adjusted to a final cell concentration of $2\times10^6$/ml in a total volume of 0.25 ml RPMI-Hepes+FCS with complement removed.

DMH, "normal liver supernatant", and Thymosine V were added at the levels indicated on Table 1. Microcytoxicity tests were performed using anti-HTLA serum and rabbit complement, after five hours incubation in a moisturized 5% $CO_2$ atmosphere.

As shown in Table 1, as low as 10 µl/ml of DMH induced acquisition of a T-cell surface marker in the null cell stock of human peripheral blood cells. The supernatant from non-conditioned liver cells was inactive.

TABLE 1

In vitro generation of HTLA[+] cells human peripheral blood cells

| | Treatment (µl/ml) | % net increase [a] | |
|---|---|---|---|
| | | Exp. 1 | Exp. 2 |
| DMH | 10 | 27.0 | 27.4 |
| | 50 | 20.5 | 18.0 |
| Liver | 10 | 2.0 | 1.0 |
| | 50 | 0 | 0 |
| Thymosin V | 50 | 5 | NT (not tested) |
| [a]Controls: | 5 to 8% | | |

(c) Differentiation induced in bone-marrow cells

T-cells and B-cells are derived from bone-marrow stem cells. It was found that DMH induces Thy-1[+] cells from bone marrow precursor cells in a variety of mouse strains, including *nu/nu* mice, to a greater extent than thymosin V, as summarized in Table 2.

Induction assays were performed as above: 2.5 hours of incubation for Thy-1 cytotoxicity assays and 6 hours of incubation at 37°C in a 5% $CO_2$ atmosphere for CR[+] B-cells, with the doses of DMH indicated in Table 2 for $5\times10^6$ bone-marrow cells.

4

TABLE 2

Effect of DMH on induction of Thy-1$^+$ on bone marrow precursor cells

| Treatment (ml/ml) | 6—8 w.old nu/nu Thy-1$^+$ | CR$^+$ | 6—8 w.old C3H/He Thy-1$^+$ | CR$^+$ | 6—8 w.old Balb/C Thy-1$^+$ | CR$^+$ | 11—12 w.old Balb/C Thy-1$^+$ | CR$^+$ | 6—8 w.old C57BK/6(2) Thy-1$^+$ | CR$^+$ |
|---|---|---|---|---|---|---|---|---|---|---|
| **DMH** 0.25 | 0 | 0 | 0 | 0 | 2 | 0 | 0 | 0 | 0 | 2 |
| 2.5 | 3.3 | 0 | 2.6 | 0.7 | 8 | 0 | 4 | 0 | 6 | 2 |
| 25 | 7.5 | 0 | 6.5 | 0 | 3 | 1 | 15 | 0 | 4 | 2 |
| 250 | — | — | 0 | 0 | 1 | 0 | 2 | 0 | 0 | 0 |
| **Thymosine** 125 | 1.5 | 2.8 | 3.5 | 1.2 | 0 | 3 | 12 | 3 | 1 | 3 |

In summary, the data in Table 2 demonstrates that on *nu/nu* spleen lymphocytes, DMH recruits T-cells from the precursor stock in bone-marrow, without modifying the B-cell lineage, however, the dose-effect curve of DMH might depend upon the strain and age of animal.

(2) *In vivo* induction of T-cell differential in *nu/nu* mice

Assays were performed by ip (intraperitoneal) administration of 0.25 ml of DMH to *nu/nu* mice (6 to 8 weeks of age). Counts for Thy-1$^+$ cells or for B cells (Ig$^+$, CR$^+$) were performed on the fourth day. Controls included untreated mice and animals treated with the supernatant of liver cells cultured without sodium diethyldithiocarbamate (DTC).

As shown in Table 3, DMH induced a high percentage of mature T-cells (Thy-1$^+$), in *nu/nu* mice, which compared favorably with the effects of known thymus hormones, inactive *in vivo*, without modifying the number of B cells, in contrast with other "hormones" (thymopietin, thymic serum factor, TFS).

TABLE 3

*In vivo* effects of DMH on induction of Thy-1 in splenocytes of *nu/nu* mice

| Treatment | % specific marker | |
| --- | --- | --- |
| | Thy-1$^+$ | Ig$^+$ |
| none | 2—5 | 24—32 |
| Liver alone | 2—6 | 26—34 |
| DMH | 15—27 | 27—34 |

G. Goldstein et al. (Science 1979, 204, 1309) described a synthetic pentapeptide, TP5, derived from thymopoietin, stated as possessing the same activities as thymopoietin.

J. F. Bach et al. (Nature, 1977, 266, 55) described FTS a nopnapeptide, stated as being the thumic hormone.

None of these prior products possesses *in vivo* activities to induce the Thy-1$^+$ phenotype in precursor cells present in the spleen of *nu/nu* mice as we show above in Table 3.

*Nu/nu* mice (C57BL/6 background) were treated with 0.1 to 10 µg/mouse DMH and the activity compared with Thymopoietin and FTS. Mice were sacrificed four days later, and Thy-1.2$^+$ and CR$^+$ cells were enumerated in the spleen (four mice per group).

TABLE 4

| Treatment (µg/mouse) | % of cells induced | |
| --- | --- | --- |
| | Thy-1$^{+*}$ | CR$^+$ |
| Saline | 0 | 33—38 |
| DMH 10 | 16—26 | 38—40 |
| 1 | 19—19 | 34—38 |
| 0.1 | 3—12 | 31—36 |
| FTS 10 | 0 | 38—39 |
| 1 | 0 | 28—39 |
| 0.1 | 0 | 26—34 |
| TP5 10 | 0 | 40—43 |
| 1 | 0 | 12—26 |
| 0.1 | 0 | 13—37 |

Background=Thy-1$^+$ cells: 2—6 (four mice per group)*=net increase

None of the doses modified the background number of CR$^+$ cells (controls: 27 to 35%), after a six hour incubation time.

A supernatant from liver cells cultured without DTC was completely unable to modify background numbers of T- or B-cells.

DMH is specifically active in recruiting T-cells from precommitted precursor cells in *nu/nu* mice, without effects on the B cell lineage, DMH was devoid of endotoxin-like products (negative *Limulus* tests).

(B) T-cell functions increased or created by DMH

1) DMH—incuded T-cell functions in *nu/nu* mice

Thymusless, *nu/nu* mice, are unable to evoke IgG-antibody-forming spleen cells (PFC) in response to immunization with SRBC.

*Nu/nu* mouse lymphocytes did not proliferate in the presence of T-cell mitogens, such as PHA or Con A.

The effects of DMH upon the T-cell incuded IgG-PFC production were studied *in vivo* by intraperitoneally administering 0.25% ml DMH at the time of iv (intravenous) immunization with $10^8$ SRBC with counts for splenic PFC made four days later (Table 5).

TABLE 5

Effect on DMH (*in vivo*) on differentiation and functional T-cell activities in *nu/nu* mice

| Test | Treatment | |
|---|---|---|
| | None (controls) | 0.25 ml DMH |
| Thy-1$^+$ cells (4) | <5 | 18±3 |
| IgG-PEC (4) | 0 | 95±7 |

The effect of DMH to modify nitrogen-incuded lymphoproliferation was studied *in vitro* by adding 0.01 ml DMH per ml culture medium ($5\times10^6$ live spleen cells) in the presence of, respectively 0.5 µg PHA, 0.5 µg Con A or 1:100 diluted PWM.

Table 6 summarized the results of a typical series of assays evidencing increased functional T-cell-mediated responses in thymusless mice treated with DMH, whereas the PWM-induced lymphoproliferation, a B-cell response in mice, was impaired.

TABLE 6

| | IgG-PFC per spleen | *In vitro* responsiveness to | | |
|---|---|---|---|---|
| | | PHA | Con A | PWM |
| DMH-treated | 95 | 1029±92 | 2604±52 | 1851±63 |
| Controls | 0 | 641±23 | 1668±38 | 2003±68 |

2) DMH elicits increased immune responses in nonresponder mice

Immunopotentiators, Levamisole and Isoprinosine (and to a lesser degree sodium diethyldithiocarbamate) are under a genetic control for responsiveness. For example, $C_3H/H3$ mice are stimulated for increased responses to SRBC, while C57BL/6 mice remain insensitive to the effect of the agents.

Table 7 shows that 0.25 ml DMH i.p. administered at the time of immunization with $10^8$ SRBC significantly stimulated PFC production in both mouse strains.

TABLE 7

Effect of DMH on SRBC antibody production

| | C3H/He | | C57BL/6 | |
|---|---|---|---|---|
| | IgM | IgH | IgM | IgG |
| Treatment | 450±170 | 51±6 | 136±58 | 17±9 |
| DMG | 1035±280 | 148±50 | 1366±270 | 53±7 |

Therefore, a treatment with DMH could be indicated in patients unresponsive to immunotherapy.

3) DMH increases Con A-induced lymphoproliferation

The effects on DMH of human peripheral blood cells (PBL) were compared with the effects of Thymosin, fraction V. In the first assay (Table 8), it was demonstrated that DMH increases the lymphoproliferation induced by suboptimal dose of Con A at a level similar to FSV (thymosin fraction V).

TABLE 8

Effect of (in vivo) DMH on Con A-induced lymphoproliferation of PBL

|  | dpm | dbd | % increase above controls |
|---|---|---|---|
| Controls | 82824 | 1212 |  |
| +20 μg FSV | 104624 | 1890 | 126 |
| +10 μg DMH | 109018 | 1470 | 132 |

Three days of culture in the presence of 20 μg of Con A. PBL, a human peripheral blood lymphocytes.

4) Suppressor activities induced by DMH

The maturing efficacy of DMH on human lymphocytes was tested by evidencing the suppressor activities of DMH-treated cells on a two-way mixed lymphocyte culture (MLC) of two HLA unrelated healthy donors. The data were compared with the data obtained with FSV-treated cells from the same donor (donor A).

TABLE 9

Effect on DMH (2 μg) on production of suppressor activity (two-way MLC) in human lymphocytes

| Blasts added ($\times 10^3$) | Cpm | % Suppression |
|---|---|---|
| 0 | 51,061 | 0 |
| 10 | 34,314 | 33 |
| 25 | 34,473 | 33 |
| 50 | 20,131 | 61 |
| 100 | 9,458 | 80 |

As shown in Table 9, DMH can induce suppressor activities against the allogenic antigen-induced lymphoproliferation at levels substantially higher than those attained by FSV-treated blast cells.

The hepatosin (DMH) of this invention has been shown to induce mature functioning T-cells from precursor cells of the immune system, thus it is an immune modulator, or immunorestorative agent.

An immunopotentiator or immunomodulator is any agent which either restores depressed immune function, or enhances normal immune function, or both. Immune function is defined as the development and expression of humoral (antibody-mediated) immunity, cellular (thymocyte-mediated) immunity, or macrophage and granulocyte mediated resistance. It logically includes agents acting directly on cellular or molecular mechanisms which, in turn, act to modify the function of cells involved in immune response. Augmentation of immune function may result from the action of an agent to abrogate suppressive mechanisms derived by negative-feedback influences endogenous or exogenous to the immune system. Thus, immune potentiators have diverse mechanisms of action. Despite the diversity of cell site of action and biochemical mechanism of action of immunopotentiators, their applications are essentially the same; that is, to enhance host resistance.

Applications of immunopotentiators

1. The principal protective function of the immune system relates to resistance to invasion by pathogens, including viruses, rickettsia, mycloplasma, bacterial fungi and parasites of all types. Thus, improvement of immune response, particularly when depressed, would calculatedly improve resistance in infection or infestation of any of the above pathogens. An immune potentiator alone or in combination with anti-infective therapy can be applied to any or all infectious diseases.

2. A second protective function of the immune system is thought to be resistance to engraftment of foreign tissue, either natural as in fetal-maternal relationship; or unnatural as performed by the transplant physician. The use of immunopotentiators to facilitate rejection of fetal or placental tissues or to modify or induce tolerance to grafts is logical.

3. A third protective function of the immune system is thought to be resistance to malignant cell

**0 058 857**

development as in cancer. The use of immunopotentiators in cancer is logical to enhance tumor rejection and to inhibit tumor recurrences following other forms of therapy.

4. A fourth protective function involves the capacity to recognize foreigness and to maintain non-reactivity to self by positive suppressor mechanisms. In auto-immune and related disorders, immune reactivity directed at self antigens or exaggerated, elevated responses are apparent which are self destructive. Immunopotentiators would logically be used to restore normal suppressor mechanisms, induce tolerance or otherwise promote a normal immune response.

Each of the protective functions of the immune system can be modified by nonspecific therapy with immunopotentiators alone or in combination with other agents employed to improve resistance or to kill the invading pathogen. In addition, specific resistance can be augmented by use of immunopotentiators in conjunction with some form of antigen as in a vaccine employing, for example, virus, tumor cell, etc. This use can to induce either specific immunity or tolerance. The latter might be exemplified by use with antigen in allergy or auto-immune disease. Use of immunopotentiators may be either therapeutic or prophylactic; the latter particularly in aging, where infection, auto-immunity, and cancer are more common. The timing of administration and routes are variable and may be critical in determining whether a positive or negative response results. Any agent capable of augmenting immune response may inhibit it depending on timing and dose; thus, under certain circumstances an immunopotentiator could be used as an immunosuppressive agent for use in allergy, auto-immunity and transplantation.

Thus the immunopotentiators of the invention can be employed, for example, to provide resistance to invasion by the viruses in the following table.

TABLE

| Virus | Class | Disease |
|---|---|---|
| Arenavirus | RNA | Rift Valley Fever |
| Influenza | RNA | Influenza |
| Rhinovirus | RNA | Common Cold |
| Poliovirus | RNA | Polio |
| Measles | RNA | Rubella |
| Newcastles Disease Virus | RNA | Newcastles disease |
| Rotavirus | RNA | Gastroenteritis in infants |
| Hepatitis Type A | RNA | Infectious Hepatitis |
| Rabies virus | RNA | Rabies |
| Arbovirus | RNA | Encephalitis |
| Vaccinia virus | DNA | Smallpox |
| Herpes Simplex Virus | DNA | Cold sore, Encephalitis, Veneral Disease |
| Herpes Zoster | DNA | Shingles |
| Varicella Zoster | DNA | Chicken pox |
| Adenovirus | DNA | Respiratory |
| Hepatitis Type B | DNA | Chronic Hepatitis, Severe Hepatitis |
| Foot and Mouth Disease Virus | DNA | Foot and Mouth Disease |
| Machupo Virus | | Hemorrhagic Fever |

The compound can also be used to treat conditions resulting from relative or absolute T-cell deficiencies such as DiGeorge Syndrome, fungal infections, mycoplasma infections, tuberculosis, leprosy, acute and chronic viral infections and systemic lupus erythemotosus.

Specifically hepatosin can be used to treat primary or secondary immunodeficiency. An example of

9

primary immunodeficiency is genetic immunodeficiencies such as severe Combined Immunodeficiency (DIC). Examples of secondary immunodeficiency are deficiencies in the immune system caused by infections (viral, bacterial, parasitic, etc.), chemotherapy (treatment of cancer), radiation therapy (cancer), surgery (any major surgery), or nutritional or environmental factors.

The compositions of the invention are useful in treating mammals (and cells of animals) including humans, swine, dogs, cats, cattle, horses, sheep, goats, mice, rabbits, rats, guinea pigs, hamsters and monkeys.

The compositions can be administered to the mammals by conventional techniques, e.g. orally, nasally, rectally, vaginally, enterally or parenterally. They can be employed as injectable solutions, e.g., in water, or as tablets, pills and capsules.

Being polypeptide in nature hepatosin can be administered by the parenteral route (im (intramuscularly), iv (intravenously), sc (subcutaneously)), and since hepatosin has a molecular weight of $<5,000$, it can be absorbed upon oral administration if protected from enzymatic degradation. The formulation for injection contains enough material to allow for administration to humans of 1—1000 µg/kg. Similar dosages can be used with the other animals.

Thus there is administered a therapeutically effective amount of the compound of the invention alone or in combination with a pharmaceutically acceptable carrier.

### Claims

1. Hepatosin, being a polypeptide-like substance with a molecular weight between 500 and 5000 and having an amino acid composition of aspartic acid, threonine, glutamic acid, glycine, cystine, tyrosine, phenylalamine and lysine and being obtainable by culturing liver cells on the medium RPMI-1640 supplemented with L-glutamine and recovering the hepatosin thus prepared.

2. A method of preparing the hepatosin of claim 1 comprising culturing liver cells on the medium RPMI-1640 supplemented with L-glutamine and recovering the hepatosin thus prepared.

3. A method according to claim 2 wherein the culture medium includes an immunopotentiator in an amount from $10^{-3}$ to $10^{-12}$ mM/ml.

4. A method according to claim 3 wherein the immunopotentiator is the sodium salt of diethyldithiocarbamic acid, levamisole or inosiplex.

5. Therapeutical composition for imparting immunomodulating activity, antiviral activity or antileukemic activity containing the hepatosin of claim 1.

6. A composition containing the hepatosin of claim 1 in a concentration of at least about 100 times as great as it is obtained by culturing $C_3H$ mouse liver cells by suspending 50 million liver cells in 50 ml of RPMI-1640 supplemented with 170 L-glutamine at 37°C for 24 hours in an atmosphere of 95% air, 5% $CO_2$, followed by harvesting by centrifugation.

7. A liquid composition containing the hepatosin of claim 1 in an amount of at least 40 mg/ml.

8. A composition according to claim 7 which is an aqueous liquid composition.

### Patentansprüche

1. Hepatosin, eine polypeptidähnliche Substanz mit eines Molekulargewicht zwischen 500 und 5000, die eine Aminosäuren-Zusammensetzung von Asparaginsäure, Threonin, Glutaminsäure, Glysin, Cystin, Tyrosin, Phenylalanin und Lysin aufweist und die gewinnbar ist durch Züchten von Leberzellen in dem Nährmedium RPMI-1640 mit Zusatz von L-Glutamin sowie Sammeln des so gebildeten Hepatosins.

2. Verfahren zur Herstellung von Hepatosin nach Anspruch 1, umfassend das Züchten von Leberzellen in dem mit L-Glutamin versehenen Nährmedium RPMI-1640 und das Sammeln des so gebildeten Hepatosins.

3. Verfahren nach Anspruch 2, wobei das Nährmedium einen Immunpotentiator in einer Menge von $10^{-3}$ bis $10^{-12}$ mM/ml enthält.

4. Verfahren nach Anspruch 3, wobei der Immunpotentiator das Natriumsalz von Diäthyldithiocarbaminsäure, Levamisol oder Inosiplex ist.

5. Therapeutische Zusammensetzung zer Herbeiführung einer immunmodulierenden, antiviralen oder antileukämischen Aktivität, enthaltend Hepatosin nach Anspruch 1.

6. Zusammensetzung enthaltend Hepatosin nach Anspruch 1 in einer wenigstens etwa 100-fach höheren Konzentration in Bezug auf eine Erzeugung durch Züchung von $C_3H$ Mauseleberzellen, mit einer Suspension von 50 Millionen Leberzellen in 50 ml mit 170 L-Glutamin versehenen Nährmediums RPMI-1640, bei 37°C während 24 Stunden in einer Atmosphäre von 95% Luft und 5% $CO_2$, gefolgt von einem Erntevorgang durch Zentrifugieren.

7. Flüssige Zusammensetzung, enthaltend Hepatosin nach Anspruch 1 in einer Menge von 40 mg/ml.

8. Zusammensetzung nach Anspruch 7 in Form einer wässrigen Flüssigkeit.

### Revendications

1. Hépatosine, en tant que substance analogue à un polypeptide avec un poids moléculaire entre 500 et 5000 et comportant une composition amino-acide d'acide aspartique, de thréonine, d'acide glutamique, de glycine, de cystine, de tyrosine, de phénylalanine et de lisine et pouvant être obtenue en cultivant des cellules de foie sur le milieu RPMI-1640 additionné de L-glutamine et en récoltant l'hépatosine ainsi obtenue.

2. Procédé pour préparer l'hépatosine de la revendication 1, comprenant la culture de cellules de foie sur le milieu RPMI-1640 additionné de L-glutamine et à récolter l'hépatosine ainsi préparés.

3. Procédé selon la revendication 2, dans lequel le milieu de culture comprend un immunopotentiateur dans une teneur de $10^{-3}$ à $10^{-12}$ mM/ml.

4. Procédé selon la revendication 3, dans lequel l'immunopotentiateur est le sel de sodium de l'acide diéthyldithiocarbamique, du levamisole ou de l'inosiplex.

5. Composition thérapeutique pour conférer une activité immunomodulaire, une activité antivirale ou une activité antileucémique contenant de l'hépatosine selon la revendication 1.

6. Composition contenant l'hépatosine selon la revendication 1, à une concentration d'au moins 100 fois cells qui avec laquelle elle est obtenue en cultivant des cellules de foie de souris $C_3H$, en suspendant 50 millions de cellules de foie dans 50 ml de RPMI-1640 additionné de 170 L-glutamine à 37°C pendant 24 heures dans une atmosphère d'air à 95% et de $CO_2$ à 5%, suivi d'une récolte par centrifugation.

7. Composition liquide contenant l'hépatosine de la revendication 1 à une concentration d'au moins 40 mg/ml.

8. Composition selon la revendication 7, qui est une composition liquide aqueuse.

FIG. 1

INDUCTION OF T-CELL MARKERS BY DMH

CONCENTRATION OF DMH
(µg/ml) BATCH #1

0 058 857

## FIG. 2

INDUCTION OF T-CELL MARKERS BY DMH

MEAN VALUE FOR 4 BATCHES (#3 TO 6) OF DMH

FIG.3    INDUCTION OF T-CELL MARKERS
BY LIVER CELL SUPERNATANT